# EUROPEAN PATENT SPECIFICATION

(11) **EP 1 882 442 B1**
(45) Date of publication and mention of the grant of the patent: **16.10.2013**
(21) Application number: 07011565.4
(22) Date of filing: 13.06.2007
(51) Int. Cl.: A61B 1/015, A61B 1/018

(54) **Fluid supply apparatus for endoscope and endoscope**
Fluidzufuhrvorrichtung für ein Endoskop und Endoskop
Appareil de distribution de liquide pour endoscope et un endoscope

(30) Priority: 24.07.2006 JP 2006200982
(43) Date of publication of application: 30.01.2008
(73) Proprietor: OLYMPUS MEDICAL SYSTEMS CORP., Tokyo 151-0072 (JP)
(72) Inventor: Suzuki, Keita, c/o Olympus Medical Systems Corp., Tokyo (JP)
(74) Representative: von Hellfeld, Axel

(56) References cited:
- WO-A-2005/044112
- DE-A1- 3 725 182
- DE-B- 1 248 221
- GB-A- 2 146 099
- US-A- 5 855 569

## Description

### BACKGROUND OF THE INVENTION

### Field of the Invention

The present invention relates to a fluid supply apparatus for an endoscope and an endoscope utilizing such a fluid supply apparatus.

### Description of Related Art

When an observation is made or medical treatment is performed using an endoscope, medical fluid or physiological saline solution or the like is supplied via a forceps channel in the endoscope. At this time, a fluid supply source such as a syringe is connected to a forceps aperture that communicates with the forceps channel either directly or via a fluid supply apparatus for an endoscope that is equipped with a valve (see, for example, Patent Document 1: Japanese Unexamined Patent Application, First Publication No. 6-304130).

However, in the above described conventional endoscope, when a treatment tool has been inserted into the forceps channel, because there is only a narrow gap between the treatment tool and the forceps channel, even if a fluid enters into the fluid channel, it is difficult for the fluid to flow through the gap and there is a possibility that it will flow in reverse through the forceps channel. Because of this, the fluid flows into a branched suction channel and, in some cases, the fluid ends up leaking to the outside through gaps in a suction button that is used to control the suction state of the endoscope.

GB 2 146 099 A relates to a device for veterinary and medical instruments such as endoscopes and bronchoscopes comprising a connection to a pump continuously drawing a vacuum or taking a suction on the system with means for bypassing the suction in full or in part. In operation, suction through the connection draws on atmosphere through a bore. This document also shows a supply tube into which either a tool or a fluid can be inserted, but not both at the same time. A movement mechanism and a valve portion are also shown.

US 5,855,569 refers to an expandable anchor mechanism for tentatively fixing a flexible tubular section of a bioptic or surgical treating instrument inserted in a biopsy channel of an endoscopic insertion rod. During a bioptic or surgical treatment, a fore end portion of the treating instrument is fixedly gripped by the anchor mechanism to stabilize a moveable operating member of the instrument which is protruded into a body cavity from the distal end of the biopsy channel. The anchor mechanism includes an anchor control means for remote-controlling an anchor member to and from an expanded state projecting into the biopsy channel to grip a treating instrument and a flatly contracted state permitting movements of the treating instrument in the biopsy channel.

### SUMMARY OF THE INVENTION

The present invention was conceived in view of the above described circumstances, and it is an object thereof to provide a fluid supply apparatus for an endoscope that is able to supply a fluid to a forceps channel even when a treatment tool has been inserted into the forceps channel, and makes it possible to suppress fluid leakage into a suction channel, as well as an endoscope that is provided with this fluid supply apparatus.

In order to solve the above described problems, the present invention employs the structure described below.

The fluid supply apparatus for an endoscope of the present invention is a fluid supply apparatus for an endoscope that is connected to an endoscope main body that has a forceps channel that has a forceps aperture in a base end and into which at least one of a treatment tool and a fluid is inserted, and a suction channel that branches from a branching portion provided partway along the forceps channel and is connected to a suction source, and includes: a mouthpiece portion having a first conduit in which is located a first aperture to which a fluid supply source is connected and a second conduit in which is located a second aperture through which the treatment tool is inserted, the mouthpiece portion adapted to connect to the forceps aperture, a supply tube portion having a flow path communicating with the first conduit and the second conduit, the flow path into which at least one of a treatment tool and fluid supplied from a fluid supply source; a movement mechanism adapted to move the supply tube portion forward inside the forceps channel when the supply tube portion is pressed in an axial direction so that a distal end portion of the supply tube portion reaches a distal end side of the forceps channel beyond the branching portion, and further adapted to move the supply tube portion backwards inside the forceps channel when the pressing state is terminated so that the supply tube portion is removed from the forceps aperture; and a valve portion provided onto an outer surface in the distal end of the supply tube portion, and adapted to block a gap formed between an outer wall surface of the supply tube portion and an inner wall surface of the forceps channel at a distal end side of the forceps channel beyond the branching portion so as to regulate the fluid flown from the supply tube portion into the forceps channel from flowing into the suction channel.

This invention makes it possible to insert a treatment tool in a supply tube portion and cause a fluid to circulate into a forceps channel through the supply tube portion by placing the supply tube portion and the forceps channel in communication with each other. At this time, circulation of the fluid to the suction channel can be regulated by the valve portion, and the fluid that has been introduced into the supply tube portion can be circulated to the forceps channel without flowing into the suction channel.

Moreover, the fluid supply apparatus for an endoscope of the present invention is a fluid supply apparatus for an endoscope in which there is further provided a mouthpiece portion that is connected to the forceps aperture, and that has a first conduit in which is located a first aperture to which the fluid supply source is connected and has a second conduit in which is located a second aperture through which the treatment tool is inserted, and in which the first aperture and the second aperture communicate with a flow path of the supply tube portion.

When a fluid is supplied from a fluid supply source to the supply tube portion, this invention makes it possible to circulate the fluid to the forceps channel irrespective of whether or not a treatment tool is present by connecting the fluid supply source to the first aperture.

Moreover, the fluid supply apparatus for an endoscope of the present invention is a fluid supply apparatus for an endoscope in which the movement mechanism is provided with a mounting portion that fixes the mouthpiece portion to the forceps aperture, and with an urging portion that urges the supply tube portion in a direction in which the supply tube portion retreats from the forceps channel relative to the mounting portion.

When the mounting portion has been fitted into the forceps aperture, this invention makes it possible to cause the supply tube portion to move forward inside the forceps channel by pressing the supply tube portion in the opposite direction from the direction in which it is urged by the urging portion. Moreover, when the supplying of the fluid has ended, the supply tube portion can be easily extracted from the forceps channel through the urging force of the urging portion.

Moreover, the fluid supply apparatus for an endoscope of the present invention is a fluid supply apparatus for an endoscope in which the movement mechanism is provided with a pressure receiving portion that receives pressure from fluid that has been supplied from the fluid supply source and causes the supply tube portion to move forward in resistance to urging force from the urging portion, and the pressure receiving portion allows the supply tube portion and the first conduit to communicate with each other in conjunction with the forward movement of the supply tube portion.

This invention makes it possible by supplying fluid to cause the supply tube portion to move forward inside a forceps channel using the pressure of the fluid, and also makes it possible to supply fluid from the first conduit to the supply tube portion.

Accordingly, it is possible to supply fluid automatically to the forceps channel.

Moreover, the fluid supply apparatus for an endoscope of the present invention is a fluid supply apparatus for an endoscope in which the supply tube portion is formed longer in the axial direction than the length from the forceps aperture to the branching portion, and in which the valve portion is a toroidal component that is located on the distal end side of the supply tube portion, and blocks off a gap that is formed between an outer wall surface of the supply tube portion and an inner wall surface on the distal end side of the forceps channel with respect to the branching portion.

When a fluid is supplied while the supply tube portion is in a state of being inserted into the forceps channel, even if fluid that has been discharged from the supply tube portion attempts to flow to the suction channel side via the gap that is formed between the outer wall surface of the supply tube portion and the inner wall surface on the distal end side of the forceps channel with respect to the branching portion, this invention makes it possible for the toroidal component to regulate movement of the fluid. Accordingly, even if there is only a small gap between a treatment tool and the forceps channel, fluid can be made to circulate through the supply tube portion to the distal end side of the forceps channel.

The endoscope of the present invention is provided with: an endoscope main body that has a forceps channel that has a forceps aperture into which a treatment tool is inserted, and a suction channel that branches at a branching portion provided partway along the forceps channel and that is connected to a suction source: and with the fluid supply apparatus for an endoscope according to the present invention.

Because this invention is provided with the fluid supply apparatus for an endoscope according to the present invention, by placing the supply tube portion and the forceps channel in communication with each other, it is possible to insert a treatment tool in the supply tube portion and to also cause a fluid to circulate to the forceps channel through the supply tube portion. At this time, the flow fluid to the suction channel can be regulated using the valve portion, and fluid that has been introduced into the supply tube portion can be made to circulate to the forceps channel without flowing into the suction channel.

According to the present invention, it is possible to supply fluid to a forceps channel and also prevent fluid leakages to a suction channel even when a treatment tool has been inserted into the forceps channel.

### BRIEF DESCRIPTION OF THE DRAWINGS

FIG 1 is an overall schematic diagram showing an endoscope having a switching valve for supplying fluid to a channel according to a first embodiment of the present invention.
FIG 2 is an explanatory diagram showing an operating state of an endoscope having a switching valve for supplying fluid to a channel according to the first embodiment of the present invention.
FIG 3 is an overall schematic diagram showing an endoscope having a switching valve for supplying fluid to a channel according to a second embodiment of the present invention.
FIG 4 is a perspective view of principal portions showing a switching valve for supplying fluid to a channel according to a second embodiment of the present invention.
FIG 5 is a cross-sectional view taken along a line A-A in FIG. 4.
FIG. 6 is an overall schematic diagram showing an endoscope having a switching valve for supplying fluid to a channel according to a third embodiment of the present invention.
FIG 7 is a cross-sectional view showing a switching valve for supplying fluid to a channel according to a third embodiment of the present invention.
FIG 8 is a cross-sectional view showing a switching valve for supplying fluid to a channel according to a third embodiment of the present invention.

### DETAILED DESCRIPTION OF THE INVENTION

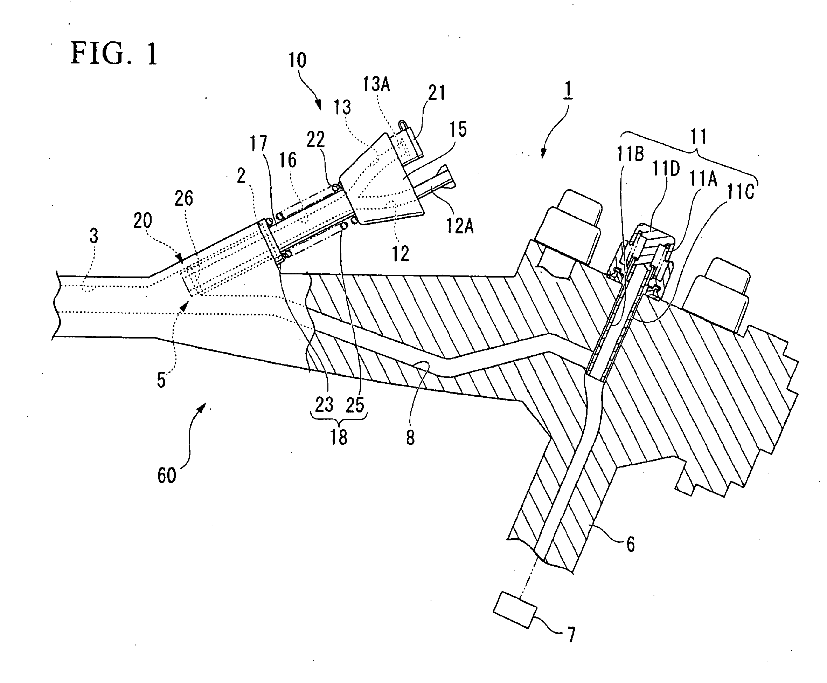

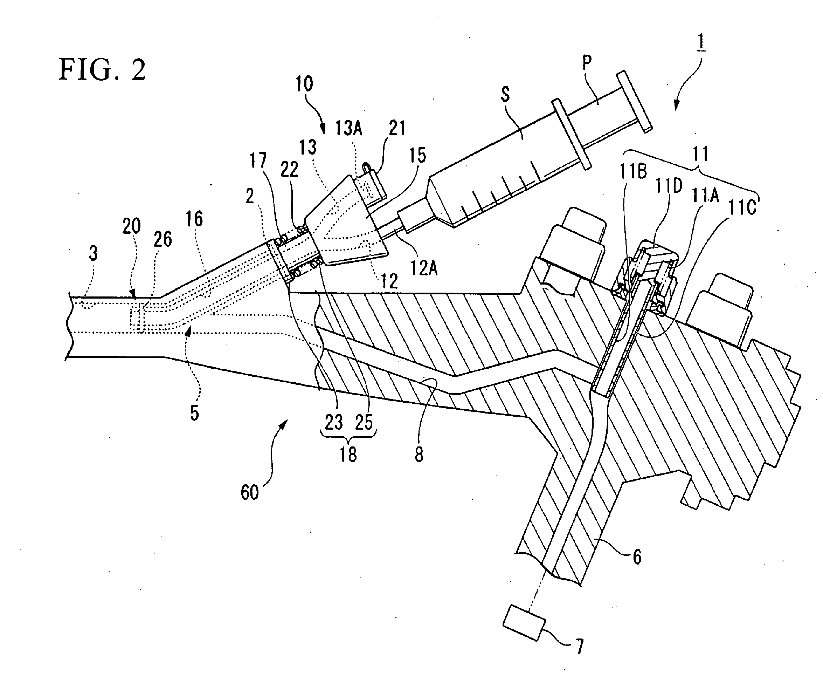

A first embodiment of the present invention will now be described using FIGS. 1 and 2.

An endoscope 1 according to the present embodiment is provided with an endoscope main body 60 that has a forceps channel 3 having a forceps aperture 2 though which a treatment tool (not shown) is inserted, and has a suction channel 8 that branches at a branching portion 5 that is provided partway along the forceps channel 3 and is connected to a suction source 7 via a universal cord 6, and with a switching valve for supplying fluid to a channel (i.e., a fluid supply apparatus for an endoscope) 10 that is connected to the endoscope main body 60, and is used to supply a fluid inside a syringe (i.e., a fluid supply source) S to the forceps channel 3. Note that the endoscope main body 60 is in a state in which the switching valve for supplying fluid to a channel 10 has been detached from the endoscope 1, and is the component that has the forceps channel 3 and the suction channel 8.

The forceps aperture 2 is provided at a base end of the forceps channel 3. A suction button 11 that controls the state of communication between the branching portion 5 and the suction source 7 is provided partway along the suction channel 8 that branches at the branching portion 5. Normally, the suction button 11 is urged by a spring portion 11A such that it regulates communication between the branching portion 5 and the suction source 7, and is also provided with a switching portion 11C that moves along a passage 11B so as to allow the branching portion 5 and the suction source 7 to communicate, and with a button portion 11 D that causes the switching portion 11C to move along the passage 11 B.

The switching valve for supplying fluid to a channel 10 is provided with: a mouthpiece portion 15 that is connected to the forceps aperture 2 and has a first conduit 12 on which is located a first aperture 12A to which the syringe S is connected and has a second conduit 13 on which is located a second aperture 13A into which a treatment tool (not shown) is inserted; a supply tube portion 17 that has a flow path 16 that communicates with the first conduit 12 and the second conduit 13; a movement mechanism 18 that, when the supply tube portion 17 is pressed in the axial direction, causes the supply tube portion 17 to move from the forceps aperture 2 into the forceps channel 3 and, when this state of being pressed is terminated, causes the supply tube portion 17 to be withdrawn in the direction in which it is removed from the forceps aperture 2; and a valve portion 20 that, as a result of the supply tube portion 17 moving forwards along the forceps channel 3, regulates any flow into the suction channel 8 of fluid flowing from the supply tube portion 17 into the forceps channel 3.

The first aperture 12A of the mouthpiece portion 15 has a shape that corresponds to the Luer fitting of the syringe S. A rubber forceps plug 21 is placed in the second aperture 13A.

The supply tube portion 17 is flexible and is connected so as to protrude from the mouthpiece portion 15.

The outer diameter of the supply tube portion 17 is formed having a size that is smaller than the mouthpiece portion 15, and enables it to be fitted via a predetermined gap into the inner diameter of the forceps channel 3. The length of the supply tube portion 17 is formed so as to be longer in the axial direction than the length of the forceps channel 3 from the forceps aperture 2 to the branching portion 5. A step portion 22 with which one end of a spring component 25 (described below) is engaged is provided in a portion where the mouthpiece portion 15 and the supply tube portion 17 are connected.

The movement mechanism 18 is provided with a mounting portion 23 that fixes the mouthpiece portion 15 in the forceps aperture 2, and the spring component (i.e., urging portion) 25 that urges the supply tube portion 17 in a direction relative to the mounting portion 23 in which it retreats from the forceps channel 3. An engaging hole (not shown) that is able to be removably engaged with the forceps aperture 2 is formed in the mounting portion 23. The spring component 25 is wound like a coil for a predetermined length around an outer surface of the supply tube portion 17 with one end of the spring component 25 being anchored by the step portion 22, and the other end thereof being connected to the mounting portion 23. The spring component 25 is wound around the outer circumference of the supply tube portion 17 in a state in which, when the syringe S is filled with fluid, the spring component 25 is compressed by a smaller force than the pressing force when the plunger P is pressed.

The valve portion 20 is provided with an O-ring (i.e., a toroidal component) 26 that is fitted onto an outer surface in the vicinity of the distal end of the supply tube portion 17, and whose outer diameter blocks a gap formed between an outer wall surface of the supply tube 17 and an inner wall surface on the distal end side of the forceps channel 3 with respect to the branching portion 5

Next, operations of the endoscope 1 and the switching valve for supplying fluid to a channel 10 of the present embodiment will be described.

Firstly, the distal end side of the supply tube portion 17 is inserted into the forceps channel 3 via the forceps aperture 2 in the endoscope 1, and the mounting portion 23 of the switching valve for supplying fluid to a channel 10 is fixed to the forceps aperture 2. The suction source 7 and the universal cord 6 are then connected so that the suction source 7 communicates with the suction channel 8.

In this state, the endoscope 1 is inserted into a body cavity and predetermined treatment is performed.

When injecting fluid from the syringe S, the syringe S that has been filled with fluid is firstly connected to the first aperture 12A. At this time, a treatment tool (not shown) is inserted through the forceps plug 21 into the forceps channel 3 via the second conduit.

In this state, the plunger P of the syringe S is pressed. At this time, the mouthpiece portion 15 moves in conjunction with the pressing of the plunger P so as to press the spring component 25 at the step portion 22 and compress the spring component 25. Note that when a fluid feed pump (not shown) is fitted to the first aperture 12A instead of the syringe S, the mouthpiece portion 15 is pushed manually into the forceps channel 3.

The supply tube portion 17 is then moved forward through the forceps channel 3 in the direction of the distal end thereof, and the distal end portion, including the O-ring 26, of the supply tube portion 17 reaches the distal end side of the forceps channel 3 beyond the branching portion 5. At this time, the gap that is formed between the supply tube portion 17 and the forceps channel 3 is sealed by the O-ring 26, so that the suction channel 8 is blocked off from the distal end side of the forceps channel 3 beyond the branching portion 5.

When the urging force of the spring component 25 reaches or exceeds the pressure of the fluid inside the syringe S which acts on the plunger P, the mouthpiece portion 15 ceases movement relative to the endoscope 1, and instead, with the spring component 25 in a compressed state, the plunger P moves in the axial direction. Because of this, the fluid inside the syringe S is discharged into the mouthpiece portion 15. This fluid flows from the first conduit 12 into the flow path 16.

At this time, even if there is only a small gap between the flow path 16 and the treatment tool, the fluid that is discharged from the supply tube portion 17 flows into the forceps channel 3 without being sidetracked into the gap between the supply tube portion 17 and the forceps channel 3. In this manner, the fluid is discharged from the forceps channel 3 onto an affected area.

When the supply of fluid is ended, by lessening the pressing force on the plunger, the spring component 25 is extended and presses against the step portion 22 so as to be restored to its original state, and the supply tube portion 17 moves inside the forceps channel 3 towards the operator side. When the supply tube portion 17 passes the branching portion 5 and the forceps channel 3 and the suction channel 8 communicate with each other once again, by pressing the suction button 11, the suction source 7 and suction channel 8 are placed in communication with each other and the forceps channel 3 is suctioned. In this manner, treatment is ended.

According to this endoscope 1 and switching valve for supplying fluid to a channel 10, by placing the supply tube portion 17 and the forceps channel 3 in communication with each other, a treatment tool can be inserted via the second conduit 13 into the supply tube portion 17, and fluid can be made to circulate from the first conduit 12 through the flow path 16 into the forceps channel 3. At this time, it is possible to regulate the circulation of fluid from the branching portion 5 to the suction channel 8 using the valve portion 20, that has been introduced from the supply tube portion 17 can be circulated just as it is to the distal end side of the forceps channel 3.

Accordingly, even when there is only a small gap between the treatment tool and the forceps channel 3 due to a treatment tool having been inserted into the forceps channel 3, or when a fluid is made to flow into the forceps channel 3 by high pressure from a pump, it is possible to supply the fluid to the forceps channel 3 while restricting the fluid from flowing into the suction channel 8. Moreover, even if a gap is formed between the suction channel 8 and the suction button 11, because the fluid does not flow into the suction channel 8, it is possible to satisfactorily prevent fluid leakages from the suction channel 8.

Moreover, when fluid is supplied from the syringe S to the supply tube portion 17, by connecting the syringe S to the first aperture 12A, it is possible to cause the fluid to circulate to the forceps channel 3 via the flow path 16 irrespective of whether or not a treatment tool is being used.

Moreover, by pressing the supply tube 17 in the opposite direction from the direction in which it is urged by the spring component 25 while the mounting portion 23 is fitted into the forceps aperture 2, the supply tube portion 17 can be made to move forward into the forceps channel 3, and fluid can be supplied simply by operating the plunger P. In addition, even if the fluid tries to flow into the suction channel 8 via the gap between the supply tube portion 17 and the forceps channel 3, because the O-ring 26 regulates movement of the fluid, the fluid can be made to circulate to the distal end side of the forceps channel 3. In contrast, when the supplying of fluid has ended, the supply tube portion 17 can be easily extracted from the forceps channel 3 by the urging force of the spring component 25.

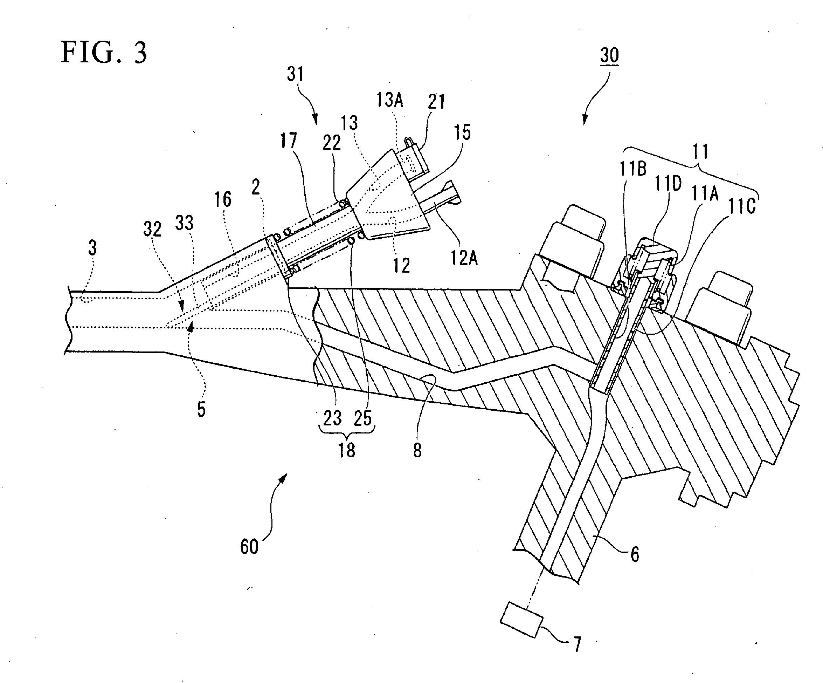

Next, a second embodiment will be described with reference made to FIG 3 through FIG 5.

Note that component elements that are the same as those in the above described first embodiment are given the same symbols and a description thereof is limited.

The second embodiment differs from the first embodiment in that, as is shown in FIG 3, a valve portion 32 of a switching valve for supplying fluid to a channel 31 that is provided in an endoscope 30 according to the present embodiment is provided with a lid component 33 instead of the O-ring 26.

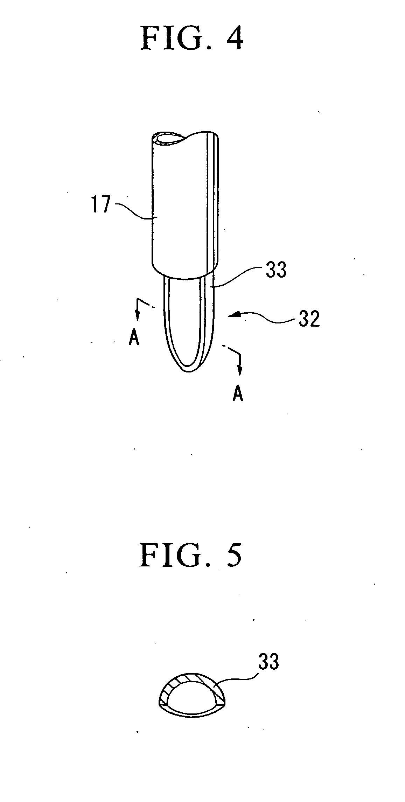

The lid component 33 is flexible and is formed as a curved surface such that, as is shown in FIG 4 and FIG 5, when it is inserted into the forceps channel 3, it protrudes on the suction channel 8 side. The lid component 33 is formed at a size that enables it to cover and seal the aperture portion of the suction channel 8 at the branching portion 5. Circumferential edge portions of the lid component 33 are connected to the distal end of the supply tube portion 17.

A description will now be given of the operation of the switching valve for supplying fluid to a channel 31 and of the endoscope 30 that is provided with this switching valve 31 when a fluid is supplied by the syringe S in the same way as in the first embodiment.

Firstly, preparations are made in the same way as in the first embodiment and the endoscope 30 is inserted into an abdominal cavity. The syringe S that has been filled with a fluid is connected to the first aperture 12A. At this time, a treatment tool (not shown) is inserted into the forceps channel 3 from the forceps plug 21 via the second conduit 13.

In this state, the plunger P of the syringe S is pressed. At this time, the mouthpiece portion 15 moves in conjunction with the pressing of the plunger P so as to press the spring component 25 and compress the spring component 25. In addition, the supply tube portion 17 is then moved forward through the forceps channel 3 in the direction of the distal end thereof, and the lid component 33 reaches the branching portion 5. At this time, the suction channel 8 is sealed at the branching portion 5 by the lid component 33, so that the suction channel 8 is closed off from the forceps channel 3.

When the urging force of the spring component 25 increases to equal or exceed the pressure of the fluid inside the syringe S which is acting on the plunger P, the plunger P moves in the axial direction with the spring component 25 in a compressed state and the fluid inside the syringe S is discharged into the mouthpiece portion 15. This fluid flows from the first conduit 12 into the flow path 16.

At this time, because the suction channel 8 is sealed at the branching portion 5 by the lid component 33, the fluid that is discharged from the supply tube portion 17 flows into the forceps channel 3 without flowing to the suction channel 8 side and flows in this state towards the distal end side. In this manner, the fluid is discharged from the forceps channel 3 onto an affected area.

According to this endoscope 30 and switching valve for supplying fluid to a channel 31, it is possible to achieve the same effects as those from the endoscope 1 and switching valve for supplying fluid to a channel 10 according to the first embodiment.

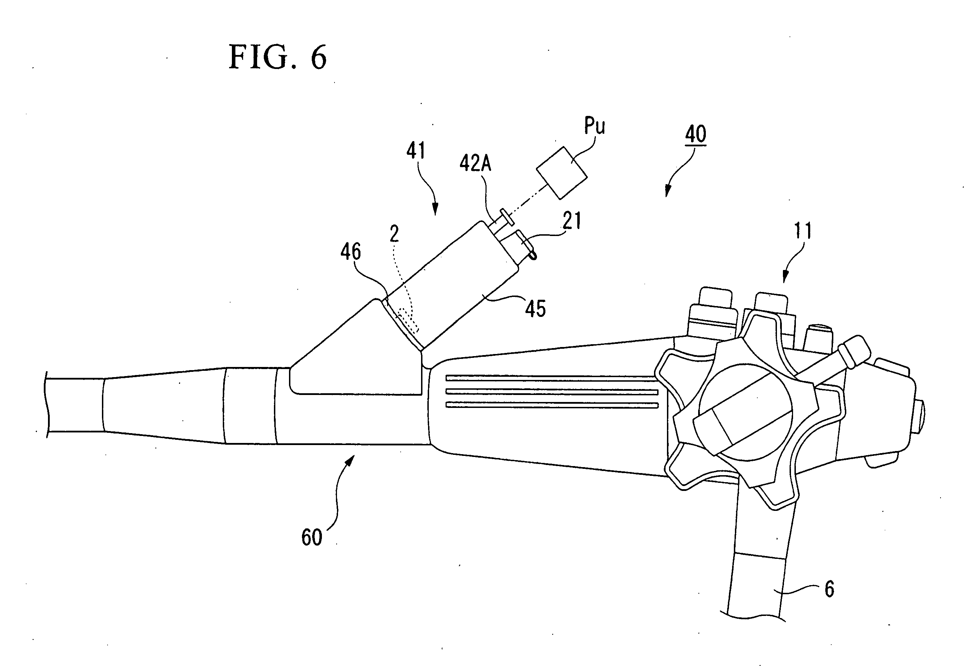

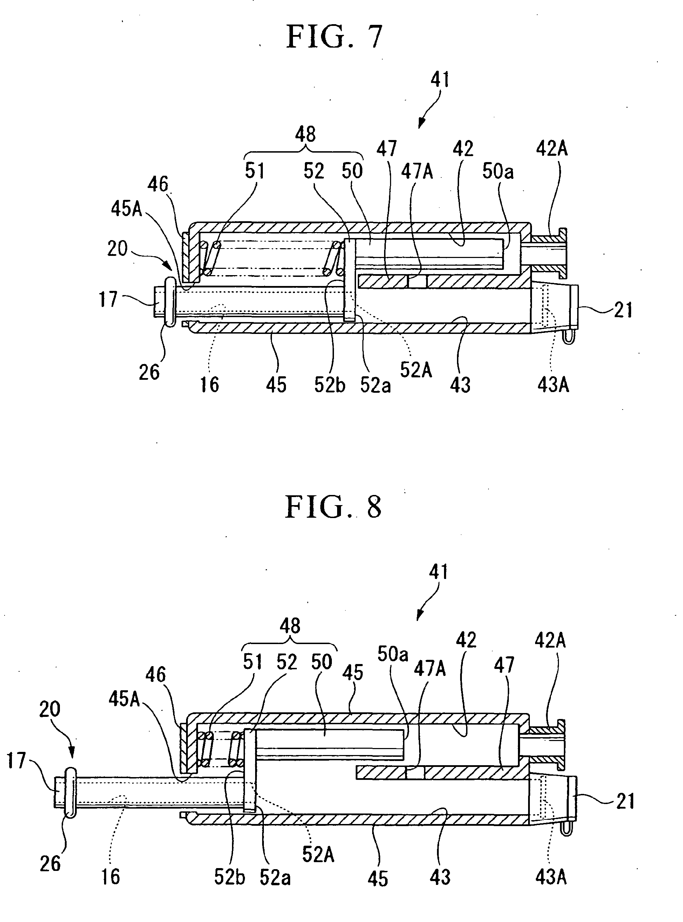

Next, a third embodiment will be described with reference made to FIG. 6 through FIG. 8.

Note that component elements that are the same as those in the above described other embodiments are given the same symbols and a description thereof is limited.

The third embodiment differs from the first embodiment in that a first conduit 42 and a second conduit 43 of a switching valve for supplying fluid to a channel 41 of an endoscope 40 according to the present embodiment are allowed to communicate with each other in accordance with a fluid discharge pressure provided by a pump (i.e., a fluid supply source) Pu that is connected to a first aperture 42A.

A mouthpiece portion 45 is formed substantially in a cylindrical shape, and the first aperture 42A and a second aperture 43A are placed at a distal end side thereof, while a mounting portion 46 that is fitted into the forceps aperture 2 is placed at a base end side thereof. The first aperture 42A has a shape that allows it to be easily connected to the pump Pu. A partition portion 47 that protrudes for a predetermined length from the distal end side towards the base end side is provided in an internal portion of the mouthpiece portion 45. The first conduit 42 and the second conduit 43 are formed by this partition portion 47. A through hole 45A through which the supply tube portion 17 can be inserted is provided in a base end surface of the mouthpiece portion 45.

The first conduit 42 and the second conduit 43 are able to communicate with each other through a side hole 47A that is provided in the partition portion 47. A piston portion 50 (described below) is fitted to the base end side of the mouthpiece portion 45 such that it is able to move in an axial direction between the distal end of the partition portion 47 and the mounting portion 46.

A movement mechanism 48 is further provided with: the piston portion 50 that is placed so as to extend to the interior of the first conduit 42 and receives pressure from the fluid that is supplied from the pump Pu so as to thereby cause the supply tube portion 17 to move; a spring component 51 that urges the piston portion 50; and a plate-shaped base portion 52 that has a first surface 52a on which the piston portion 50 is standing, and has a second surface 52b to which the spring component 51 is connected.

The piston portion 50 has substantially the same outer diameter as the inner diameter of the first conduit 42, and is fitted into the first conduit 42 via a predetermined gap. The length of the piston portion 42 is formed so as to allow the first conduit 42 and the second conduit 43 to be in communication with each other at the side hole 47A when the base portion 52 has moved the maximum amount possible to the mounting portion 46 side while pressing the spring component 51. The distal end of the piston portion 50 forms a pressure receiving surface (i.e., a pressure receiving portion) 50a that receives pressure from fluid that has been introduced through the first aperture 42A. Unlike the first embodiment, the spring component 51 is placed inside the mouthpiece portion 45 without being wound onto the supply tube portion 17, and one end thereof is connected to a second surface 52b of the base portion 52 while the other end thereof is connected to the mounting portion 46. A through hole 52A that allows the second conduit 43 and the flow path 16 to communicate with each other is provided in the base portion 52.

Next, operations of the endoscope 40 and the switching valve for supplying fluid to a channel 41 according to the present embodiment will be described.

Firstly, preparations are made in the same way as in the first embodiment and the endoscope 40 is inserted into an abdominal cavity and the pump Pu is connected to the first aperture 42A. At this time, a treatment tool (not shown) is inserted from the forceps plug 21 into the interior of the forceps channel 3 via the second conduit 43.

In this state, fluid is supplied from the pump Pu. At this time, the piston portion 50 is pressed via the pressure receiving surface 50a by pressure from the fluid that has been injected through the first aperture 42A into the first conduit 42, and the base 52 moves from the distal end side to the base end side while pressing the spring component 51 so as to compress the spring component 51. The tube supply portion 17 is then moved forward through the forceps channel 3 in the direction of the distal end thereof, and the distal end portion including the O-ring 26 reaches the distal end side of the forceps channel 3 beyond the branching portion 5. At this time, the gap that is formed between the supply tube portion 17 and the forceps channel 3 is sealed by the O-ring 26, so that the suction channel 8 is blocked off from the forceps channel 3.

As a result of the base portion 52 then moving further, the distal end of the piston portion 50 passes through the side hole 47A so that the first conduit 42 and the second conduit 43 are in communication with each other. In this manner, the fluid inside the first conduit 42 passes through the second conduit 43 and is discharged from the through hole 52A into the flow path 16. Here, in the same way as in the first embodiment, because the gap between the supply tube portion 17 and the forceps channel 3 is sealed by the O-ring 26, the fluid that is discharged from the supply tube portion 17 flows into the forceps channel 3 without being sidetracked into the gap between the supply tube portion 17 and the forceps channel 3. In this manner, the fluid is discharged from the forceps channel 3 onto an affected area.

According to this endoscope 40 and switching valve for supplying fluid to a channel 41, it is possible to achieve the same effects as those from the endoscope 1 and switching valve for supplying fluid to a channel 10 according to the first embodiment. In particular, by supplying fluid from the pump Pu, it is possible to cause the supply tube portion 17 to move forward inside the forceps channel 3 using the pressure from the pump Pu, and it is possible to supply fluid from the first conduit 42 to the supply tube portion 17. Accordingly, fluid can be supplied automatically to the forceps channel 3.

Note that the technology range of the present invention is not limited to the above described embodiments and various modifications may be made thereto insofar as they do not depart from the scope of the present invention.

For example, in the above described embodiments, the endoscope main body is separate from the switching valve for supplying fluid to a channel, however, it is also possible for them to be formed as a single unit and connected to the forceps aperture.

## Claims

1. A fluid supply apparatus for an endoscope that is connected to an endoscope main body (60) that has a forceps channel (3) that has a forceps aperture (2) in a base end and into which at least one of a treatment tool and a fluid is inserted, and a suction channel (8) that branches from a branching portion (5) provided partway along the forceps channel (3) and is connected to a suction source (7), wherein the fluid supply apparatus comprises:
a mouthpiece portion (15) having a first conduit (12) in which is located a first aperture (12A) to which a fluid supply source (S) is connected and a second conduit (13) in which is located a second aperture (13A) through which the treatment tool is inserted, the mouthpiece portion (15) adapted to connect to the forceps aperture (2);
a supply tube portion (17) having a flow path (16) communicating with the first conduit (12) and the second conduit (13), the flow path (16) into which at least one of a treatment tool and fluid supplied from the fluid supply source (S) is inserted;
a movement mechanism (18) adapted to move the supply tube portion (17) forward inside the forceps channel (3) when the supply tube portion (17) is pressed in an axial direction so that a distal end portion of the supply tube portion (17) reaches a distal end side of the forceps channel (3) beyond the branching portion (5), and further adapted to move the supply tube portion (17) backwards inside the forceps channel (3) when the pressing state is terminated so that the supply tube portion (17) is removed from the forceps aperture (2); and
a valve portion (20) provided on an outer surface at the distal end of the supply tube portion (17), and adapted to block a gap formed between an outer wall surface of the supply tube portion (17) and an inner wall surface of the forceps channel (3) at a distal end side of the forceps channel (3) beyond the branching portion (5) so as to regulate the fluid flown from the supply tube portion (17) into the forceps channel (3) from flowing into the suction channel (8).

2. The fluid supply apparatus for an endoscope according to claim 1, wherein the movement mechanism (18) is provided with a mounting portion (23) that fixes the mouthpiece portion (15) to the forceps aperture (2), and an urging portion (22) that urges the supply tube portion (17) in a direction in which the supply tube portion (17) retreats from the forceps channel (3) relative to the mounting portion (23).

3. The fluid supply apparatus for an endoscope according to claim 2, wherein the movement mechanism (18) is provided with a pressure receiving portion (50a) that receives pressure from fluid that has been supplied from the fluid supply source (S) and causes the supply tube portion (17) to move forward in resistance to urging force from the urging portion (22), and
the pressure receiving portion (50a) allows the supply tube portion (17) and the first conduit (12) to communicate with each other in conjunction with the forward movement of the supply tube portion (17).

4. The fluid supply apparatus for an endoscope according to claim 1, wherein the supply tube portion (17) is formed longer in the axial direction than the length from the forceps aperture (2) to the branching portion (5), and
the valve portion (20) is a toroidal component that is located on the distal end side of the supply tube portion (17), and blocks off the gap that is formed between an outer wall surface of the supply tube portion (17) and an inner wall surface of the forceps channel (3) on the distal end side of the branching portion (5).

5. An endoscope comprising:
an endoscope main body (60) that has a forceps channel (3) that has a forceps aperture (2) into which a treatment tool is inserted, and a suction channel (8) that branches at a branching portion (5) provided partway along the forceps channel (3) and that is connected to a suction source (7); and
the fluid supply apparatus for an endoscope according to one of claims 1 to 4.

## Patentansprüche

1. Fluidzuführeinrichtung für ein Endoskop, die mit einem Endoskop-Hauptteil (60) verbunden ist, das einen Zangenkanal (3), der in einem Basisende eine Zangenöffnung (2) aufweist und in den ein Behandlungswerkzeug und/oder ein Fluid eingebracht wird, und einen Saugkanal (8) aufweist, der von einem auf halbem Weg entlang des Zangenkanals (3) vorgesehenen Abzweigbereich (5) abzweigt und mit einer Saugquelle (7) verbunden ist, wobei die Fluidzuführeinrichtung aufweist:
ein Mundstückteil (15) mit einem ersten Kanal (12), in dem sich eine erste Öffnung (12A) befindet, an die eine Fluidzuführquelle (S) angeschlossen ist, und einem zweiten Kanal (13), in dem sich eine zweite Öffnung (13A) befindet, durch die das Behandlungswerkzeug eingeführt wird, wobei das Mundstückteil (15) mit der Zangenöffnung (2) verbunden zu werden vermag;
einen Zuführrohrbereich (17) mit einem mit dem ersten Kanal (12) und dem zweiten Kanal (13) in Verbindung stehenden Strömungsweg (16), in den das Behandlungswerkzeug und/oder das von der Fluidzuführquelle (S) zugeführte Fluid eingebracht wird;
einen Bewegungsmechanismus (18), der dazu ausgebildet ist, den Zuführrohrbereich (17) im Inneren des Zangenkanals (3) nach vorne zu bewegen, wenn der Zuführrohrbereich (17) in axialer Richtung mit Druck beaufschlagt wird, so dass ein distaler Endabschnitt des Zuführrohrbereichs (17) eine jenseits des Abzweigbereichs (5) gelegene distale Endseite des Zangenkanals (3) erreicht, und der ferner dazu ausgebildet ist, den Zuführrohrbereich (17) im Inneren des Zangenkanals (3) nach hinten zu bewegen, wenn der Zustand der Druckbeaufschlagung beendet ist, so dass der Zuführrohrbereich (17) von der Zangenöffnung (2) zurückgezogen wird; und
einen Ventilbereich (20), der an einer Außenfläche an dem distalen Ende des Zuführrohrbereichs (17) vorgesehen ist und dazu ausgebildet ist, einen zwischen einer Außenwandoberfläche des Zuführrohrbereichs (17) und einer Innenwandoberfläche des Zangenkanals (3) gebildeten Spalt an einer distalen Endseite des Zangenkanals (3) jenseits des Abzweigbereichs (5) abzusperren, um das von dem Zuführrohrbereich (17) in den Zangenkanal (3) geflossene Fluid vom Fließen in den Saugkanal (8) abzuriegeln.

2. Fluidzuführeinrichtung für ein Endoskop nach Anspruch 1, wobei der Bewegungsmechanismus (18) einen das Mundstückteil (15) an der Zangenöffnung (2) fixierenden Anbringungsbereich (23) und einen Druckbeaufschlagungsbereich (22) aufweist, der den Zuführrohrbereich (17) in eine Richtung drückt, in der sich der Zuführrohrbereich (17) relativ zum Anbringungsbereich (23) gesehen von dem Zangenkanal (3) zurückzieht.

3. Fluidzuführeinrichtung für ein Endoskop nach Anspruch 2, wobei der Bewegungsmechanismus (18) einen Druckaufnahmebereich (50a) aufweist, der Druck von dem von der Fluidzuführquelle (S) zugeführten Fluid aufnimmt und den Zuführrohrbereich (17) zu einer Vorwärtsbewegung gegen den Widerstand der von dem Druckbeaufschlagungsbereich (22) aufgebrachten Druckkraft veranlasst, und
der Druckaufnahmebereich (50a) dem Zuführrohrbereich (17) und dem ersten Kanal (12) erlaubt gemäß der Vorwärtsbewegung des Zuführrohrbereichs (17) miteinander in Verbindung zu treten.

4. Fluidzuführeinrichtung für ein Endoskop nach Anspruch 1, wobei der Zuführrohrbereich (17) so ausgebildet ist, dass seine Länge in axialer Richtung größer ist als die Länge von der Zangenöffnung (2) zum Abzweigbereich (5), und
wobei der Ventilbereich (20) ein Rundringelement ist, das an der distalen Endseite des Zuführrohrbereichs (17) positioniert ist und den zwischen einer Außenwandoberfläche des Zuführrohrbereichs (17) und einer Innenwandoberfläche des Zangenkanals (3) gebildeten Spalt an der distalen Endseite des Abzweigbereichs (5) absperrt.

5. Endoskop, das aufweist:
ein Endoskop-Hauptteil (60), das einen Zangenkanal (3) mit einer Zangenöffnung (2), in die ein Behandlungswerkzeug eingebracht wird, und einen Saugkanal (8) aufweist, der von einem auf halbem Weg entlang des Zangenkanals (3) vorgesehenen Abzweigbereich (5) abzweigt und mit einer Saugquelle (7) verbunden ist; und
die Fluidzuführeinrichtung für ein Endoskop nach einem der Ansprüche 1 bis 4.

## Revendications

1. Appareil d'alimentation en liquide pour un endoscope qui est connecté à un corps principal d'endoscope (60) qui comporte un canal de pince (3) qui comporte une ouverture de pince (2) dans une extrémité de base et dans lequel au moins l'un parmi un outil de traitement et un liquide est inséré, et un canal d'aspiration (8) qui bifurque d'une partie d'embranchement (5) prévue à mi-chemin le long du canal de pince (3) et est connecté à une source d'aspiration (7), dans lequel l'appareil d'alimentation en liquide comprend :
une partie d'embouchure (15) comportant un premier conduit (12) dans lequel est placée une première ouverture (12A) à laquelle une source d'alimentation en liquide (S) est connectée et un deuxième conduit (13) dans lequel est placée une deuxième ouverture (13A) à travers laquelle l'outil de traitement est inséré, la partie d'embouchure (15) étant adaptée pour se connecter à l'ouverture de pince (2) ;
une partie de tube d'alimentation (17) comportant un trajet d'écoulement (16) communiquant avec le premier conduit (12) et le deuxième conduit (13), le trajet d'écoulement (16) dans lequel au moins l'un parmi un outil de traitement et un liquide délivré depuis la source d'alimentation en liquide (S) est inséré ;
un mécanisme de déplacement (18) adapté pour déplacer la partie de tube d'alimentation (17) vers l'avant à l'intérieur du canal de pince (3) lorsque la partie de tube d'alimentation (17) est enfoncée dans la direction axiale de sorte qu'une partie d'extrémité distale de la partie de tube d'alimentation (17) atteint un côté d'extrémité distale du canal de pince (3) au-delà de la partie d'embranchement (5), et en outre adapté pour déplacer la partie de tube d'alimentation (17) vers l'arrière à l'intérieur du canal de pince (3) lorsque l'état d'enfoncement est terminé de sorte que la partie de tube d'alimentation (17) est enlevée de l'ouverture de pince (2) ; et
une partie de clapet (20) prévue sur une surface externe au niveau de l'extrémité distale de la partie de tube d'alimentation (17), et adaptée pour bloquer un espace formé entre une surface de paroi externe de la partie de tube d'alimentation (17) et une surface de paroi interne du canal de pince (3) au niveau d'un côté d'extrémité distale du canal de pince (3) au-delà de la partie d'embranchement (5) de façon à empêcher que le liquide s'écoulant de la partie de tube d'alimentation (17) dans le canal de pince (3) entre dans le canal d'aspiration (8).

2. Appareil d'alimentation en liquide pour un endoscope selon la revendication 1, dans lequel le mécanisme de déplacement (18) est muni d'une partie de montage (23) qui fixe la partie d'embouchure (15) à l'ouverture de pince (2), et d'une partie de sollicitation (22) qui sollicite la partie de tube d'alimentation (17) dans une direction, dans laquelle la partie de tube d'alimentation (17) se retire du canal de pince (3) par rapport à la partie de montage (23).

3. Appareil d'alimentation en liquide pour un endoscope selon la revendication 2, dans lequel le mécanisme de déplacement (18) est muni d'une partie de réception de pression (50a) qui reçoit une pression provenant du liquide qui a été délivré depuis la source d'alimentation en liquide (S) et amène la partie de tube d'alimentation (17) à se déplacer vers l'avant en résistant à la force de sollicitation provenant de la partie de sollicitation (22), et
la partie de réception de pression (50a) permet que la partie de tube d'alimentation (17) et le premier conduit (12) communiquent l'un avec l'autre en liaison avec le déplacement vers l'avant de la partie de tube d'alimentation (17).

4. Appareil d'alimentation en liquide pour un endoscope selon la revendication 1, dans lequel la partie de tube d'alimentation (17) est formée plus longue dans la direction axiale que la longueur allant de l'ouverture de pince (2) à la partie d'embranchement (5), et
la partie de clapet (20) est un composant toroïdal qui est placé sur le côté d'extrémité distale de la partie de tube d'alimentation (17), et barre l'espace qui est formé entre une surface de paroi externe de la partie de tube d'alimentation (17) et une surface de paroi interne du canal de pince (3) sur le côté d'extrémité distale de la partie d'embranchement (5).

5. Endoscope comprenant :
un corps principal d'endoscope (60) qui comporte un canal de pince (3) qui comporte une ouverture de pince (2) dans laquelle un outil de traitement est inséré, et un canal d'aspiration (8) qui bifurque au niveau d'une partie d'embranchement (5) prévue à mi-chemin le long du canal de pince (3) et qui est connecté à une source d'aspiration (7) ; et
l'appareil d'alimentation en liquide pour un endoscope selon l'une quelconque des revendications 1 à 4.
